# EUROPEAN PATENT APPLICATION

(11) **EP 1 602 726 A2**
(43) Date of publication of application: **07.12.2005**
(21) Application number: 05010911.5
(22) Date of filing: 02.05.2002
(51) Int. Cl.: C12N 15/12, C07K 14/47, C07K 7/22, A61K 38/17

(54) **Human tachykinin-related splice variants and compositions thereof**

(30) Priority: 01.05.2001 US 288214 P
(62) Divisional of application: 02750880.3
(71) Applicant: Geneprot, Inc., 1217 Meyrin 2 (CH)
(72) Inventor: Bougueleret, Lydie, 1213 Petit-Lancy (CH); Kappus, Stephanie, 1208 Geneva (CH)
(74) Representative: Vossius & Partner

(57) **Abstract**

The invention provides an TSV polypeptide, methods and compositions for making such peptide, and methods of using the polypeptide and agonists and antagonists thereof for treating phosphate wasting disorders.

SEQUENCE LISTING

<110> Bougueleret, Lydie
      Kappus, Stephanie
<120> Human Tachykinin-Related Splice Variants and Compositions Thereof
<130> 5003
<140>
<141> 2001-05-01
<160> 3
<170> Microsoft Word 2000

<210> 1

<211> 129

<212> PRT

<213> Homo Sapiens

<400> 1

Met Lys Ile Leu Val Ala Leu Ala Val Phe Phe Leu Val Ser Thr Gln
                    5                   10
15

Leu Phe Ala Glu Glu Ile Gly Ala Asn Asp Asp Leu Asn Tyr Trp Ser
                20                  25                  30

Asp Trp Tyr Asp Ser Asp Gln Ile Lys Glu Glu Leu Pro Glu Pro Phe
            35                  40                  45

Glu His Leu Leu Gln Arg Ile Ala Arg Arg Pro Lys Pro Gln Gln Phe
    50                  55                  60

Phe Gly Leu Met Gly Lys Arg Asp Ala Asp Ser Ser Ile Glu Lys Gln
65                  70                  75
80

Val Ala Leu Leu Lys Ala Leu Tyr Gly His Gly Gln Ile Ser His Lys
                    85                  90
95

Arg His Lys Thr Asp Ser Phe Val Gly Leu Met Gly Lys Arg Ala Leu
                100                 105                 110

Asn Ser Val Ala Tyr Glu Arg Ser Ala Met Gln Asn Tyr Glu Arg Arg
        115                 120                 125

Arg

<210> 2

<211> 36

<212> PRT

<213> Homo Sapiens

<400> 2

Asp Ala Asp Ser Ser Ile Glu Lys Gln Val Ala Leu Leu Lys Ala Leu
                5                   10                  15

Tyr Gly His Gly Gln Ile Ser His Lys Arg His Lys Thr Asp Ser Phe
            20                  25                  30

Val Gly Leu Met
        35

<210>  3

<211>  28

<212>  PRT

<213>  Homo Sapiens

<400>  3

Asp Ala Asp Ser Ser Ile Glu Lys Gln Val Ala Leu Leu Lys Ala Leu
                5                   10                  15

Tyr Gly His Lys Thr Asp Ser Phe Val Gly Leu Met
            20                  25

## Description

### Field of the Invention

The invention relates generally to secreted low molecular weight human proteins, and more particularly, to polypeptides and other compositions related to the human tachykinin family of proteins, and uses thereof.

### BACKGROUND

Many low molecular weight secreted proteins have profound effects both in health and disease, either by growth stimulating roles, growth inhibitory roles, or the regulation of critical metabolic pathways. Such molecules include growth factors, cytokines, peptide hormones, and like compounds. Growth factors are proteins that bind to receptors on cell surfaces, with the primary result of activating cellular proliferation or differentiation. Many growth factors are pleiotropic, stimulating cell division or other effects in numerous different cell types; while others are specific to a particular cell type or tissue. Many growth factors or products derived from them have become important medicines, such as erythropoietin (EPO), interferon-α (αINF), and granulocyte macrophage colony stimulating factor (GM-CSF); and many others, e.g. insulin-like growth factor-1 (IGF-1), tumor growth factor-α (TGF-α), interleukins, fibroblast growth factor proteins, and others, are under intensive study to undertand their roles in a variety of diseases, particularly cancer, e.g. Jameson, pp. 73-82, in Jameson, ed., Principles of Molecular Medicine (Humana Press, Totowa, NJ, 1998).

The tachykinins are a family of neuropeptides that have a variety of biological activities, including activities related to the generation of pain and neurological damage following hippocampal seizures, e.g. Liu et al, Proc. Natl. Acad. Sci., 96: 12096-12101 (1999); Zimmer et al, Proc. Natl. Acad. Sci., 95: 2630-2635 (1998); Cao et al, Nature, 392: 390-394 (1998). The expression levels of tachykinin-related polypeptides are regulated at the transcriptional and post-translational stages of synthesis.

The availability of the active tachykinin polypeptide and related compounds for enhancing or otherwise modulating the biological effects of tachykinin would satisfy a need in the art by providing new therapeutic strategies for managing pain or seizures.

### SUMMARY OF THE INVENTION

The present invention is directed to compositions related to a human tachykinin splice variant (TSV) polypeptide, antibodies specific for TSV polypeptide, and methods of making and using such compositions. The invention further includes methods of using TSV polypeptide compositions, including antibody compounds, to treat disorders associated aberrant expression of TSV polypeptide in an individual.

In one aspect, the invention includes polypeptides having an amino acid sequence with at least 97 percent identity with the sequence selected from the group consisting of SEQ ID NO: 2 and SEQ ID NO: 3. More preferably, the invention includes polypeptides having an amino acid sequence with at least 97 percent identity with the sequence selected from the group consisting of SEQ ID NO: 2. Most preferably, the invention includes a polypeptide having an amino acid sequence identical to SEQ ID NO: 2.

In another aspect, the invention includes an isolated peptide consisting of 6 to 30 amino acids whose sequence is identical to a subsequence of consecutive amino acids in the polypeptide of SEQ ID NO: 2. Such peptides are useful intermediates in the production of antigenic compositions used in the production of peptide antibodies specific for TSV polypeptide.

In another aspect, the invention includes isolated antibodies specific for any of the polypeptides, peptide fragments, or peptides described above. Preferably, the antibodies of the invention are monoclonal antibodies. Such antibodies have diagnostic and therapeutic applications, particularly in treating TSV polypeptide-related disorders. Treatment methods include, but are not limited to, those that employ antibodies or antibody-derived compositions specific for an TSV polypeptide antigen. Diagnostic methods for detecting an TSV polypeptide in specific tissue samples, and for detecting levels of expression of an TSV polypeptide in tissues, also form part of the invention.

In another aspect, the invention includes an isolated polynucleotide that encodes TSV polypeptide of SEQ ID NO: 2.

In another aspect, the invention includes natural variants of the TSV polypeptide having a frequency in a selected population of at least two percent. More preferably, such natural variant has a frequency in a selected population of at least five percent, and most preferably, of at least ten percent. The selected population may be any recognized population of study in the field of population genetics. Preferably, the selected population is Caucasian, Negroid, or Asian. More preferably, the selected population is French, German, English, Spanish, Swiss, Japanese, Chinese, Korean, Singaporean of Chinese ancestry, Icelandic, North American, Israeli, Arab, Turkish, Greek, Italian, Polish, Pacific Islander, or Indian.

In another aspect, the invention provides a vector comprising DNA encoding a TSV polypeptide. The invention also includes host cells comprising such a vector. A process for producing a TSV polypeptide is also provided which comprises culturing the host cells under conditions suitable for expression of such TSV polypeptide and its recovery from the cell culture materials.

In still a further aspect, the invention includes pharmaceutical compositions and formulations comprising a polypeptide having an amino acid sequence of SEQ ID NO: 2 and a pharmaceutically acceptable carrier compound.

### Brief Description of the Figures

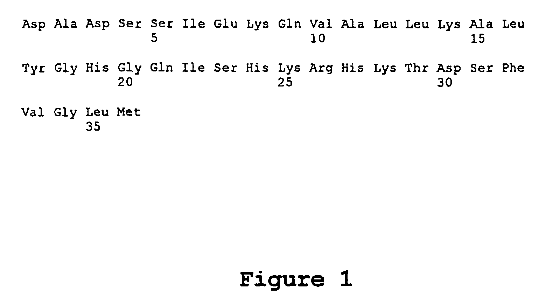

Figure 1 is a listing of the amino acid sequence of the human TSV polypeptide of the invention.

### DEFINITIONS

The terms "polypeptide" or "peptide" or "peptide fragment" as used herein refers to a compound made up of a single unbranched chain of amino acid residues linked by peptide bonds. The number of amino acid residues in such compounds varies widely; however, preferably, peptides referred to herein usually have from six to forty amino acid residues. Polypeptides and peptide fragments referred to herein usually have from a few tens of amino acid residues, e.g. 20, to up to a few hundred amino acid residues, e.g. 200, or more. Generally, polypeptides are manufactured more conveniently by recombinant DNA methods.

The term "protein" as used herein may be used synonymously with the term "polypeptide" or may refer to, in addition, a complex of two or more polypeptides which may be linked by bonds other than peptide bonds, for example, such polypeptides making up the protein may be linked by disulfide bonds. The term "protein" may also comprehend a family of polypeptides having identical amino acid sequences but different post-translational modifications, such as phosphorylations, acylations, glycosylations, and the like, particularly as may be added when such proteins are expressed in eukaryotic hosts.

Amino acid residues are referred to herein by their standard single-letter or three-letter notations: A, alanine; C, cysteine; D, aspartic acid; E, glutamic acid; F, phenylalanine; G, glycine; H, histidine; I, Isoleucine; K, lysine; L, leucine; M, methionine; N, asparagine; P, proline; Q, glutamine; . R, arginine; S, serine; T, threonine; V, valine; W, tryptophan; Y, tyrosine.

"Perfectly matched" in reference to a duplex means that the poly- or oligonucleotide strands making up the duplex form a double stranded structure with one other such that every nucleotide in each strand undergoes Watson-Crick basepairing with a nucleotide in the other strand. The term also comprehends the pairing of nucleoside analogs, such as deoxyinosine, nucleosides with 2-aminopurine bases, and the like, that may be employed. In reference to a triplex, the term means that the triplex consists of a perfectly matched duplex and a third strand in which every nucleotide undergoes Hoogsteen or reverse Hoogsteen association with a basepair of the perfectly matched duplex. Conversely, a "mismatch" in a duplex between a tag and an oligonucleotide means that a pair or triplet of nucleotides in the duplex or triplex fails to undergo Watson-Crick and/or Hoogsteen and/or reverse Hoogsteen bonding.

The term "percent identical," or like term, used in respect of the comparison of a reference sequence and another sequence (i.e. a "candidate" sequence) means that in an optimal alignment between the two sequences, the candidate sequence is identical to the reference sequence in a number of subunit positions equivalent to the indicated percentage, the subunits being nucleotides for polynucleotide comparisons or amino acids for polypeptide comparisons. As used herein, an "optimal alignment" of sequences being compared is one that maximizes matches between subunits and minimizes the number of gaps employed in constructing an alignment. Percent identities may be determined with commercially available implementations of algorithms described by Needleman and Wunsch, J. Mol. Biol., 48: 443-453 (1970)("GAP" program of Wisconsin Sequence Analysis Package, Genetics Computer Group, Madison, WI). Other software packages in the art for constructing alignments and calculating percentage identity or other measures of similarity include the "BestFit" program, based on the algorithm of Smith and Waterman, Advances in Applied Mathematics, 2: 482-489 (1981) (Wisconsin Sequence Analysis Package, Genetics Computer Group, Madison, WI). In other words, for example, to obtain a polypeptide having an amino acid sequence at least 95 percent identical to a reference amino acid sequence, up to five percent of the amino acid residues in the reference sequence many be deleted or substituted with another amino acid, or a number of amino acids up to five percent of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence of in one or more contiguous groups with in the references sequence. It is understood that in making comparisons with reference sequences of the invention that candidate sequence may be a component or segment of a larger polypeptide or polynucleotide and that such comparisons for the purpose computing percentage identity is to be carried out with respect to the relevant component or segment.

The term "isolated" in reference to a polypeptide or polynucleotide of the invention means that the indicated polypeptide or polynucleotide has been separated from the components of its natural environment.

The term "oligonucleotide" as used herein means linear oligomers of natural or modified monomers or linkages, including deoxyribonucleosides, ribonucleosides, anomeric forms thereof, peptide nucleic acids (PNAs), and the like, capable of specifically binding to a polynucleotide by way of a regular pattern of monomer-to-monomer interactions, such as Watson-Crick type of base pairing, base stacking, Hoogsteen or reverse Hoogsteen types of base pairing, or the like. Usually, monomers are linked by phosphodiester bonds, or analogs thereof, to form oligonucleotides ranging in size from a few monomeric units, e.g. 3-4, to several tens of monomeric units, e.g. 40-60. Whenever an oligonucleotide or polynucleotide is represented by a sequence of letters, such as "ATGCCTG," or the lower case equivalent, it will be understood that the nucleotides are in 5'→3' order from left to right and that "A" denotes deoxyadenosine, "C" denotes deoxycytidine, "G" denotes deoxyguanosine, "T" denotes thymidine, and "U" denotes uridine, unless otherwise noted or understood for their context. Usually oligonucleotides of the invention comprise the four natural nucleotides, and they are joined to one another by natural phosphodiester linkages; however, they may also comprise non-natural nucleotide analogs and may also contain non-natural inter-nucleosidic linkages, particularly when employed as antisense or diagnostic compositions. It is clear to those skilled in the art when oligonucleotides having natural or non-natural nucleotides may be employed in accordance with the invention, e.g. where processing by enzymes is called for, usually oligonucleotides consisting of natural nucleotides are required.

As used herein, "nucleoside" includes the natural nucleosides, including 2'-deoxy and 2'-hydroxyl forms, e.g. as described in Kornberg and Baker, DNA Replication, 2nd Ed. (Freeman, San Francisco, 1992). "Analogs" in reference to nucleosides includes synthetic nucleosides having modified base moieties and/or modified sugar moieties, e.g. described by Scheit, Nucleotide Analogs (John Wiley, New York, 1980); Uhlman and Peyman, Chemical Reviews, 90: 543-584 (1990), or the like, with the only proviso that they are capable of specific hybridization. Such analogs include synthetic nucleosides designed to enhance binding properties, reduce complexity, increase specificity, and the like.

### DETAILED DESCRIPTION OF THE INVENTION

The present invention encompasses TSV polypeptides and related compositions of matter including, but not limited to, polynucleotides encoding TSV polypeptide or fragments thereof, antibodies specific for TSV polypeptide or fragments thereof, recombinant DNA constructs and vectors comprising polynucleotides of the invention as well as host cells containing such constructs or vectors used for replicating TSV transcripts or for expressing TSV polypeptides. The invention also encompasses pharmaceutical compositions comprising TSV polypeptide, and agonists and antagonists thereof, particularly antagonists derived from monoclonal antibodies specific for TSV polypeptide compositions.

TSV polypeptide and peptide fragments of the invention include natural and man-made variants whose amino acid sequences differ from the reference amino acid sequences of the Sequence Listing by one or more substitutions, insertions, or deletions. Such variants ordinarily are prepared by site specific mutagenesis of nucleotides in the DNA encoding the TSV polypeptide or peptide fragment, using cassette or PCR mutagenesis or other techniques well known in the art, to produce DNA encoding the variant, and thereafter expressing the DNA in recombinant cell culture, as described more fully below. Variant TSV polypeptides may also be synthesized chemically using conventional peptide synthesis techniques or convergent synthesis techniques as described below

Natural variants of the polypeptides of the invention are obtained by conventional screening of individuals of a selected population using analysis techniques employing oligonucleotides of the invention. Preferably, genomic regions containing all or a portion of a genomic region is amplified using PCR or like technique, after which the amplified sequence is sequenced using conventional methods, or otherwise analyzed at specific loci using conventional techniques. The sequence is then compared to polynucleotides of the invention to determine whether a variation affecting the encoded protein is present. Preferably, natural TSV polypeptide variants of the invention have a frequency in the population of two percent or greater, and more preferably, of five percent or greater, and most preferably, of ten percent or greater.

### Recombinant Manufacture of TSV polypeptide

The polynucleotide sequences described herein can be used in recombinant DNA molecules that direct the expression of the corresponding polypeptides in appropriate host cells. Because of the degeneracy in the genetic code, other DNA sequences may encode the equivalent amino acid sequence, and may be used to clone and express the TSV polypeptides. Codons preferred by a particular host cell may be selected and substituted into the naturally occurring nucleotide sequences, to increase the rate and/or efficiency of expression. The nucleic acid (e.g., cDNA or genomic DNA) encoding the desired TSV polypeptide may be inserted into a replicable vector for cloning (amplification of the DNA), or for expression. The polypeptide can be expressed recombinantly in any of a number of expression systems according to methods known in the art (Ausubel, et al., editors, Current Protocols in Molecular Biology, John Wiley & Sons, New York, 1990). Appropriate host cells include yeast, bacteria, archebacteria, fungi, and insect and animal cells, including mammalian cells, for example primary cells, including stem cells, including, but not limited to bone marrow stem cells. More specifically, these include, but are not limited to, microorganisms such as bacteria transformed with recombinant bacteriophage, plasmid or cosmid DNA expression vectors, and yeast transformed with yeast expression vectors. Also included, are insect cells infected with a recombinant insect virus (such as baculovirus), and mammalian expression systems. The nucleic acid sequence to be expressed may be inserted into the vector by a variety of procedures. In general, DNA is inserted into an appropriate restriction endonuclease site using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The TSV polypeptides of the present invention are produced by culturing a host cell transformed with an expression vector containing a nucleic acid encoding a TSV polypeptide, under the appropriate conditions to induce or cause expression of the protein. The conditions appropriate for TSV polypeptide expression will vary with the choice of the expression vector and the host cell, and will be easily ascertained by one skilled in the art through routine experimentation. For example, the use of constitutive promoters in the expression vector will require optimizing the growth and proliferation of the host cell, while the use of an inducible promoter requires the appropriate growth conditions for induction. In addition, in some embodiments, the timing of the harvest is important. For example, the baculoviral systems used in insect cell expression are lytic viruses, and thus harvest time selection can be crucial for product yield.

A host cell strain may be chosen for its ability to modulate the expression of the inserted sequences or to process the expressed protein in the desired fashion. Such modifications of the protein include, but are not limited to, acetylation, carboxylation, glycosylation, phosphorylation, lipidation and acylation. Post-translational processing, which cleaves a "prepro" form of the protein, may also be important for correct insertion, folding and/or function. By way of example, host cells such as CHO, HeLa, BHK, MDCK, 293, W138, etc. have specific cellular machinery and characteristic mechanisms for such post-translational activities and may be chosen to ensure the correct modification and processing of the introduced, foreign protein. Of particular interest are *Drosophila melangastev* cells, *Sacchavomyces cevevisiae* and other yeasts, *E. coli, Bacillus subtilis*, SF9 cells, C129 cells, 293 cells, Neurospora, BHK, CHO, COS, and HeLa cells, fibroblasts, Schwanoma cell lines, immortalized mammalian myeloid and lymphoid cell lines, Jukat cells, human cells and other primary cells.

The nucleic acid encoding an TSV polypeptide must be "operably linked" by placing it into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" DNA sequences are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice. Promoter sequences encode either constitutive or inducible promoters. The promoters may be either naturally occurring promoters or hybrid promoters. Hybrid promoters, which combine elements of more than one promoter, are also known in the art, and are useful in the present invention. The expression vector may comprise additional elements, for example, the expression vector may have two replication systems, thus allowing it to be maintained in two organisms, for example in mammalian or insect cells for expression and in a procaryotic host for cloning and amplification. Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2: plasmid origin is suitable for yeast, and various viral origins (SV40, polyoma, adenovirus, VSV or BPV) are useful for cloning vectors in mammalian cells. Further, for integrating expression vectors, the expression vector contains at least one sequence homologous to the host cell genome, and preferably, two homologous sequences which flank the expression construct. The integrating vector may be directed to a specific locus in the host cell by selecting the appropriate homologous sequence for inclusion in the vector. Constructs for integrating vectors are well known in the art.

Preferably, the expression vector contains a selectable marker gene to allow the selection of transformed host cells. Selection genes are well known in the art and will vary with the host cell used. Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available for from complex media, e.g., the gene encoding D-alanine racemase for Bacilli.

Host cells transformed with a nucleotide sequence encoding a prostate tumor antigen may be cultured under conditions suitable for the expression and recovery of the encoded protein from cell culture. The protein produced by a recombinant cell may be secreted, membrane-bound, or contained intracellularly depending on the sequence and/or the vector used. As will be understood by those of skill in the art, expression vectors containing polynucleotides encoding the TSV polypeptide can be designed with signal sequences which direct secretion of the TSV polypeptide through a prokaryotic or eukaryotic cell membrane. The desired TSV polypeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the TSV polypeptide-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including Saccharomyces and Kluyveromyces a-factor leaders, the latter described in U.S. Pat. No. 5,010,182), or acid phosphatase leader, the *C. albicans* glucoamylase leader (EP 362,179 published Apr. 4, 1990), or the signal described in WO 90113646 published Nov. 15, 1990. In mammalian cell expression, mammalian signal sequences may be used to direct secretion of the protein, such as signal sequences from secreted polypeptides of the same or related species, as well as viral secretory leaders. According to the expression system selected, the coding sequence is inserted into an appropriate vector, which in turn may require the presence of certain characteristic "control elements" or "regulatory sequences." Appropriate constructs are known generally in the art (Ausubel, et al., 1990) and, in many cases, are available from commercial suppliers such as Invitrogen (San Diego, Calif.), Stratagene (La Jolla, Calif.), Gibco BRL (Rockville, Md.) or Clontech (Palo Alto, Calif.).

Expression in Bacterial Systems. Transformation of bacterial cells may be achieved using an inducible promoter such as the hybrid lacZ promoter of the "BLUESCRIPT" Phagemid (Stratagene) or "pSPORT1" (Gibco BRL). In addition, a number of expression vectors may be selected for use in bacterial cells to produce cleavable fusion proteins that can be easily detected and/or purified, including, but not limited to "BLUESCRIPT" (a-galactosidase; Stratagene) or pGEX (glutathione S-transferase; Promega, Madison, Wis.). A suitable bacterial promoter is any nucleic acid sequence capable of binding bacterial RNA polymerase and initiating the downstream (3') transcription of the coding sequence of the TSV polypeptide gene into mRNA. A bacterial promoter has a transcription initiation region which is usually placed proximal to the 5' end of the coding sequence. This transcription initiation region typically includes an RNA polymerase binding site and a transcription initiation site. Sequences encoding metabolic pathway enzymes provide particularly useful promoter sequences. Examples include promoter sequences derived from sugar metabolizing enzymes, such as galactose, lactose and maltose, and sequences derived from biosynthetic enzymes such as tryptophan. Promoters from bacteriophage may also be used and are known in the art. In addition, synthetic promoters and hybrid promoters are also useful; for example, the tat promoter is a hybrid of the trp and lac promoter sequences. Furthermore, a bacterial promoter can include naturally occurring promoters of non-bacterial origin that have the ability to bind bacterial RNA polymerase and initiate transcription. An efficient ribosome binding site is also desirable. The expression vector may also include a signal peptide sequence that provides for secretion of the prostate tumor antigen protein in bacteria. The signal sequence typically encodes a signal peptide comprised of hydrophobic amino acids which direct the secretion of the protein from the cell, as is well known in the art. The protein is either secreted into the growth media (gram-positive bacteria) or into the periplasmic space, located between the inner and outer membrane of the cell (gram-negative bacteria). The bacterial expression vector may also include a selectable marker gene to allow for the selection of bacterial strains that have been transformed. Suitable selection genes include drug resistance genes such as ampicillin, chloramphenicol, erythromycin, kanamycin, neomycin and tetracycline. Selectable markers also include biosynthetic genes, such as those in the histidine, tryptophan and leucine biosynthetic pathways. When large quantities of TSV polypeptides are needed, e.g., for the induction of antibodies, vectors which direct high level expression of fusion proteins that are readily purified may be desirable. Such vectors include, but are not limited to, multifunctional *E. coli* cloning and expression vectors such as BLUESCRIPT (Stratagene), in which the prostate tumor antigen coding sequence may be ligated into the vector in-frame with sequences for the amino-terminal Met and the subsequent 7 residues of beta-galactosidase so that a hybrid protein is produced; PIN vectors [Van Heeke & Schuster *JBiol Chem 264:5503-5509* 1989)]; PET vectors (Novagen, Madison Wis.); and the like. Expression vectors for bacteria include the various components set forth above, and are well known in the art. Examples include vectors for *Bacillus subtilis, E. coli, Streptococcus cvemovis,* and *Streptococcus lividans*, among others. Bacterial expression vectors are transformed into bacterial host cells using techniques well known in the art, such as calcium chloride mediated transfection, electroporation, and others.

Expression in Yeast. Yeast expression systems are well known in the art, and include expression vectors for *Sacchavomyces cevevisiae, Candida albicans* and *C. maltosa, Hansenula polymovpha, Kluyvevomyces fvagilis* and *K. lactis, Pichia guillevimondii* and *P pastoris, Schizosaccha-vomyces pombe,* and *Yawowia lipolytica*. Examples of suitable promoters for use in yeast hosts include the promoters for 3-phosphoglycerate kinase [Hitzeman et al., *J. Biol. Chem.* 255:2073 (1980)] or other glycolytic enzymes [Hess et al.,J. *Adv. Enzyme Reg.* 7:149 (1968); Holland, *Biochemistry* 17:4900 (1978)], such as enolase, glyceraldehyde-3- phosphate dehydrogenase, hexokinase, pyruvate decarboxylase, phosphofructokinase, glucose- 6-phosphate isomerase, 3-phosphoglycerate mutase, pyruvate kinase, tri osephosphate isomerase, phosphoglucose isomerase, alpha factor, the ADH2IGAPDH promoter, glucokinase alcohol oxidase, and PGH. [See, for example, Ausubel, et al., 1990; Grant et al., *Methods in Enzymology* 153:516-544, (1987)]. Other yeast promoters, which are inducible have the additional advantage of transcription controlled by growth conditions, include the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors andpromoters for use in yeast expression are further described in EP 73,657. Yeast selectable markers include ADE2. HIS4. LEU2. TRPI. and ALG7, which confers resistance'to tunicamycin; the neomycin phosphotransferase gene, which confers resistance to G418; and the CUPI gene, which allows yeast to grow in the presence of copper ions. Yeast expression vectors can be constructed for intracellular production or secretion of a TSV polypeptide from the DNA encoding the TSV polypeptide of interest. For example, a selected signal peptide and the appropriate constitutive or inducible promoter may be inserted into suitable restriction sites in the selected plasmid for direct intracellular expression of the TSV polypeptide. For secretion of the TSV polypeptide, DNA encoding the TSV polypeptide can be cloned into the selected plasmid, together with DNA encoding the promoter, the yeast alpha-factor secretory signal/leader sequence, and linker sequences (as needed), for expression of the TSV polypeptide. Yeast cells, can then be transformed with the expression plasmids described above, and cultured in an appropriate fermentation media. The protein produced by such transformed yeast can then be concentrated by precipitation with 10% trichloroacetic acid and analyzed following separation by SDS-PAGE and staining of the gels with Coomassie Blue stain. The recombinant TSV polypeptide can subsequently be isolated and purified from the fermentation medium by techniques known to those of skill in the art.

Expression in Mammalian Systems. The TSV polypeptides may be expressed in mammalian cells. Mammalian expression systems are known in the art, and include retroviral vector mediated expression systems. Mammalian host cells may be transformed with any of a number of different viral-based expression systems, such as adenovirus, where the coding region can be ligated into an adenovirus transcription/translation complex consisting of the late promoter and tripartite leader sequence. Insertion in a nonessential E1 or E3 region of the viral genome results in a viable virus capable of expression of the polypeptide of interest in infected host cells. A preferred expression vector system is a retroviral vector system such as is generally described in PCT/US97/01019 and PCT/US97/101048. Suitable mammalian expression vectors contain a mammalian promoter which is any DNA sequence capable of binding mammalian RNA polymerase and initiating the downstream (3') transcription of a coding sequence for TSV polypeptide into mRNA. A promoter will have a transcription initiating region, which is usually placed proximal to the 5' end of the coding sequence, and a TATA box, using a located 25-30 base pairs upstream of the transcription initiation site. The TATA box is thought to direct RNA polymerase II to begin RNA synthesis at the correct site. A mammalian promoter will also contain an upstream promoter element (enhancer element), typically located within 100 to 200 base pairs upstream of the TATA box. An upstream promoter element determines the rate at which transcription is initiated and can act in either orientation. Of particular use as mammalian promoters are the promoters from mammalian viral genes, since the viral genes are often highly expressed and have a broad host range. Examples include promoters obtained from the genomes of viruses such as polyoma virus, fowlpox virus (UK 2,211, 504 published Jul. 5,1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems. Transcription of a DNA encoding a TSV polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, a-fetoprotein, and insulin). Typically, however, one will use an enhancer from a eukaryotic cell virus. Examples include the SV40 enhancer, the cytomegalovirus early promoter enhancer, the polyoma enhancer on the late side of the replication origin, and adenovirus enhancers. The enhancer is preferably located at a site 5' from the promoter. In general, the transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-translational cleavage and polyadenylation. Examples of transcription terminator and polyadenylation signals include those derived from SV40. Long term, high-yield production of recombinant proteins can be effected in a stable expression system. Expression vectors which contain viral origins of replication or endogenous expression elements and a selectable marker gene may be used for this purpose. Appropriate vectors containing selectable markers for use in mammalian cells are readily available commercially and are known to persons skilled in the art. Examples of such selectable markers include, but are not limited to herpes simplex virus thymi-dine kinase and adenine phosphoribosyltransferase for use in tk- or hprt-cells, respectively. The methods of introducing exogenous nucleic acid into mammalian hosts, as well as other hosts, is well known in the art, and will vary with the host cell used. Techniques include dextran-mediated transfection, calcium phosphate precipitation, polybrene mediated transfection, protoplast fusion, electroporation, viral infection, encapsulation of the polynucleotide(s) in liposomes, and direct microinjection of the DNA into nuclei.

Expression in Insect Cells. TSV polypeptides may also be produced in insect cells. Expression vectors for the transformation of insect cells, and in particular, baculovirus-based expression vectors, are well known in the art. In one such system, the TSV polypeptide-encoding DNA is fused upstream of an epitope tag contained within a baculovirus expression vector. *Autographa califovnica* nuclear polyhedrosis virus (AcNPV) is used as a vector to express foreign genes in *Spodoptevu fmgipevdu* Sf9 cells or in Trichoplusia larvae. The TSV polypeptide-encoding sequence is cloned into a nonessential region of the virus, such as the polyhedrin gene, and placed under control of the polyhedrin promoter. Successful insertion of a TSV polypeptide-encoding sequence will render the polyhedrin gene inactive and produce recombinant virus lacking coat protein coat. The recombinant viruses are then used to infect *S. fmgipevdu* cells or Trichoplusia larvae in which the TSV polypeptide is expressed [Smith et al., J. Wol. 46:584 (1994); Engelhard E K et al., *Pvoc. Nat. Acad. Sci.* 91:3224-3227 (1994)]. Suitable epitope tags for fusion to the TSV polypeptide-encoding DNA include poly-his tags and immunoglobulin tags (like Fc regions of IgG). A variety of plasmids may be employed, including commercially available plasmids such as pVL1393 (Novagen). Briefly, the TSV polypeptide-encoding DNA or the desired portion of the TSV polypeptide-encoding DNA is amplified by PCR with primers complementary to the 5' and 3' regions. The 5' primer may incorporate flanking restriction sites. The PCR product is then digested with the selected restriction enzymes and subcloned into an expression vector. Recombinant baculovirus is generated by co-transfecting the above plasmid and BaculoGoldTM virus DNA (Phamungen) into *Spodopteva fvugipevda* ("Sf9") cells (ATCC CRL 1711) using lipofectin (commercially available from GIBCO-BRL), or other methods known to those of skill in the art. Virus is produced by day 4-5 of culture in Sf9 cells at 28°C., and used for further amplifications. Procedures are performed as further described in O'Reilley et al., *BACULOVIRUS EXPRESSION VECTORS: A LABORATORY MANUAL*, Oxford University Press (1994). Extracts may be prepared from recombinant virus-infected Sf9 cells as described in Rupert et al., *Nature* 362:175-179 (1993). Alternatively, expressed epitope-tagged TSV polypeptides can be purified by affinity chromatography, or for example, purification of an IgG tagged (or Fc tagged) TSV polypeptide can be performed using chromatography techniques, including Protein A or protein G column chromatography.

Evaluation of Gene Expression. Gene expression may be evaluated in a sample directly, for example, by standard techniques known to those of skill in the art, e.g., Southern blotting for DNA detection, Northern blotting to determine the transcription of mRNA, dot blotting (DNA or RNA), or in situ hybridization, using an appropriately labeled probe, based on the sequences provided herein. Alternatively, antibodies may be used in assays for detection of nucleic acids, such as specific duplexes, including DNA duplexes, RNA duplexes, and DNA-RNA hybrid duplexes or DNA-protein duplexes. Such antibodies may be labeled and the assay carried out where the duplex is bound to a surface, so that upon the formation of duplex on the surface, the presence of antibody bound to the duplex can be detected. Gene expression, alternatively, may be measured by immunohistochemical staining of cells or tissue sections and assay of cell culture or body fluids, to directly evaluate the expression of TSV polypeptides. Antibodies useful for such immunological assays may be either monoclonal or polyclonal, and may be prepared against a native sequence TSV polypeptide based on the DNA sequences provided herein.

Purification of Expressed Protein. Expressed TSV polypeptides may be purified or isolated after expression, using any of a variety of methods known to those skilled in the art. The appropriate technique will vary depending upon what other components are present in the sample. Contaminant components that are removed by isolation or purification are materials that would typically interfere with diagnostic or therapeutic uses for the polypeptide, and may include enzymes, hormones, and other solutes. The purification step(s) selected will depend, for example, on the nature of the production process used and the particular TSV polypeptide produced. An TSV polypeptide or protein may be recovered from culture medium or from host cell lysates. If membrane-bound, it can be released from the membrane using a suitable detergent solution (e.g. Triton-X 100) or by enzymatic cleavage. Alternatively, cells employed in expression of TSV polypeptides can be disrupted by various physical or chemical means, such as freeze-thaw cycling, sonication, mechanical disruption, or by use of cell lysing agents. Exemplary purification methods include, but are not limited to, ion-exchange column chromatography; chromatography using silica gel or a cation-exchange resin such as DEAE; gel filtration using, for example, Sephadex G-75; protein A Sepharose columns to remove contaminants such as IgG; chromatography using metal chelating columns to bind epitope-tagged forms of the TSV polypeptide; ethanol precipitation; reverse phase HPLC; chromatofocusing; SDS-PAGE; and ammonium sulfate precipitation. Ordinarily, an isolated TSV polypeptide will be prepared by at least one purification step. For example, the TSV polypeptide may be purified using a standard anti-TSV polypeptide antibody column. Ultrafiltration and dialysis techniques, in conjunction with protein concentration, are also useful (see, for example, Scopes, R., *PROTEIN PURIFICATION,* Springer-Verlag, New York, N.Y., 1982). The degree of purification necessary will vary depending on the use of the TSV polypeptide. In some instances no purification will be necessary. Once expressed and purified as needed, the TSV polypeptides and nucleic acids of the present invention are useful in a number of applications, as detailed below.

Labeling of Expressed Protein. The nucleic acids, proteins and antibodies of the invention may be labeled. By labeled herein is meant that a compound has at least one element, isotope or chemical compound attached to enable the detection of the compound. In general, labels fall into three classes: a) isotopic labels, which may be radioactive or heavy isotopes; b) immune labels, which may be antibodies or antigens; and c) colored or fluorescent dyes. The labels may be incorporated into the compound at any position that does not interfere with the biological activity or characteristic of the compound which is being detected.

TSV polypeptide Fusion Proteins. The TSV polypeptide of the present invention may also be modified in a way to form chimeric molecules comprising a TSV polypeptide fused to another, heterologous polypeptide or amino acid sequence. The term "fusion protein" used herein refers to a chimeric polypeptide comprising a TSV polypeptide, or domain sequence thereof, fused to a "targeting polypeptide". The targeting polypeptide has enough residues to facilitate targeting to a particular cell type or receptor, yet is short enough such that it does not interfere with the biological function of the TSV polypeptide. The targeting polypeptide preferably is also fairly unique so that the fusion protein does not substantially cross-react with other cell types or receptors. Suitable targeting polypeptides generally have at least about 10 amino acid residues and usually between from about 10 to about 500 amino acid residues. Preferred targeting polypeptides have from about 20 to about 200 amino acid residues. The fusion protein may also comprises a fusion of a TSV polypeptide with a tag polypeptide which provides an epitope to which an anti-tag antibody can selectively bind. The epitope tag is generally placed at the amino-or carboxyl-terminus of the TSV polypeptide. Such epitope-tagged forms of an TSV polypeptide can be detected using an antibody against the tag polypeptide. Also, provision of the epitope tag enables the TSV polypeptide to be readily purified by using an anti-tag antibody or another type of affinity matrix that binds to the epitope tag. Alternatively, the fusion protein may comprise a fusion of a TSV polypeptide with an immunoglobulin or a particular region of an immunoglobulin. For a bivalent form of the chimeric molecule, such a fusion could be to the Fc region of an IgG molecule or, for example, GM-CSF. Preferred fusion proteins include, but are not limited to, molecules that facilitate immune targeting of the TSV polypeptide. The TSV polypeptide fusion protein may be made for various other purposes using techniques well known in the art. For example, for the creation of antibodies, if the desired epitope is small, a partial or complete TSV polypeptide may be fused to a carrier protein to form an immunogen. Alternatively, the TSV polypeptide may be made as a fusion protein to increase the ability of the antigen to stimulate cellular and/or humoral (antibody-based) immune responses, or for other reasons.

Synthetic Genes for TSV polypeptides. Once nucleic acid sequence and/or amino acid sequence information is available for a native protein a variety of techniques become available for producing virtually any mutation in the native sequence, e.g.Shortle, in Science, Vol. 229, pgs. 1193-1201 (1985); Zoller and Smith, Methods in Enzymology, Vol. 100, pgs. 468-500 (1983); Mark et al., U.S. Patent 4,518,584; Wells et al., in Gene, Vol. 34, pgs. 315-323 (1985); Estell et al., Science, Vol. 233, pgs. 659-663 (1986); Mullenbach et 20 al., J. Biol. Chem., Vol. 261, pgs. 719-722 (1986), and Feretti et al., Proc. Natl. Acad. Sci., Vol. 83, pgs.. 597-603 (1986). Accordingly, these references are incorporated by reference.

Variants of the natural polypeptide (sometime referred to as "muteins") may be desirable in a variety of circumstances. For example, undesirable side effects might be reduced by certain variants, particularly if the side effect activity is associated with a different part of the polypeptide from that of the desired activity. In some expression systems, the native polypeptide may be susceptible to degradation by proteases. In such cases, selected substitutions and/or deletions of amino acids which change the susceptible sequences can significantly enhance yields, e.g. British patent application 2173-804-A where Arg at position 275 of human tissue plasminogen activator is replaced by Gly or Glu. Variants may also increase yields in purification procedures and/or increase shelf lives of proteins by eliminating amino acids susceptible to oxidation, acylation, alkylation, or other chemical modifications. For example, methionines readily undergo oxidation to form sulfoxides, which in many proteins is associated with loss of biological activity, e.g. Brot and Weissbach, Arch. Biochem. Biophys., Vol. 223, pg. 271 (1983). Often methionines can be replaced by more inert amino acids with little or no loss of biological activity, e.g. Australian patent application AU-A-52451/86. In bacterial expression systems, yields can sometimes be increased by eliminating or replacing conformationally inessential cystiene residues, e.g. Mark et al., U.S. Patent 4,518,584.

Preferably cassette mutagenesis is employed to generate mutant proteins. A synthetic gene is constructed with a sequence of unique (when inserted in an appropriate vector) restriction endonuclease sites spaced approximately uniformly along the gene. The unique restriction sites allow segments of the gene to be conveniently excised and replaced with synthetic oligonucleotides (i.e. "cassettes") which code for desired mutations. Determination of the number and distribution of unique restriction sites entails the consideration of several factors including (1) preexisting restriction sites in the vector to be employed in expression, (2) whether species or genera-specific codon usage is desired, (3) the number of different non-vector-cutting restriction endonucleases available (and their multiplicities within the synthetic gene), and (4) the convenience and reliability of synthesizing and/or sequencing the segments between the unique restriction sites.

The above technique is a convenient way to effect conservative amino acid substitutions, and the like, in the native protein sequence. "Conservative" as used herein means (i) that the alterations are as conformationally neutral as possible, that is, designed to produce minimal changes in the tertiary structure of the mutant polypeptides as compared to the native protein, and (ii) that the alterations are as antigenically neutral as possible, that is, designed to produce minimal changes in the antigenic determinants of the mutant polypeptides as compared to the native protein.. The following is a preferred categorization of amino acids into similarity classes: aromatic (phe, trp, tyr), hydrophobic (leu, ile, val), polar (gln, asn), basic (arg, lys, his), acidic (asp, glu), small (ala, ser, thr, met, gly). Conformational neutrality is desirable for preserving biological activity, and antigenic neutrality is desirable for avoiding the triggering of immunogenic responses in patients or animals treated with the compounds of the invention. While it is difficult to select with absolute certainty which alternatives will be conformationally and antigenically neutral, rules exist which can guide those skilled in the art to make alterations that have high probabilities of being conformationally and antigenically neutral, e.g. Anfisen (cited above); Berzofsky, Science, Vol. 229, pgs. 932-940 (1985); and Bowie et al, Science, Vol. 247, pgs. 1306-1310 (1990). Some of the more important rules include (1) substitution of hydrophobic residues are less likely to produce changes in antigenicity because they are likely to be located in the protein's interior, e.g. Berzofsky (cited above) and Bowie et al (cited above); (2) substitution of physiochemically similar, i.e. synonymous, residues are less likely to produce conformational changes because the replacement amino acid can play the same structural role as the substituted amino acid; and (3) alteration of evolutionarily conserved sequences is likely to produce deleterious conformational effects because evolutionary conservation suggests sequences may be functionally important. In addition to such basic rules for selecting variant sequences, assays are available to confirm the biological activity and conformation of the engineered molecules. Biological assays for the polypeptides of the invention are described more fully in the cited references. Changes in conformation can be tested by at least two well known assays: the microcomplement fixation method, e.g. Wasserman et al., J. Immunol., Vol. 87, pgs. 290-295 (1961), or Levine et al. Methods in Enzymology, Vol. 11, pgs. 928-936 (1967) used widely in evolutionary studies of the tertiary structures of proteins; and affinities to sets of conformation-specific monoclonal antibodies, e.g. Lewis et al., Biochemistry, Vol. 22, pgs. 948-954 (1983).

### Chemical Manufacture of TSV polypeptide

Peptides of the invention are synthesized by standard techniques, e.g. Stewart and Young, Solid Phase Peptide Synthesis, 2nd Ed. (Pierce Chemical Company, Rockford, IL, 1984). Preferably, a commercial peptide synthesizer is used, e.g. Applied Biosystems, Inc. (Foster City, CA) model 430A, and polypeptides of the invention may be assembled from multiple, separately synthesized and purified, peptide in a convergent synthesis approach, e.g. Kent et al, U.S. patent 6,184,344 and Dawson and Kent, Annu. Rev. Biochem., 69: 923-960 (2000). Peptides of the invention are assembled by solid phase synthesis on a cross-linked polystyrene support starting from the carboxyl terminal residue and adding amino acids in a stepwise fashion until the entire peptide has been formed. The following references are guides to the chemistry employed during synthesis: Merrifield, J. Amer. Chem. Soc., Vol. 85, pg. 2149 (1963); Kent et al., pg 185, in Peptides 1984, Ragnarsson, Ed. (Almquist and Weksell, Stockholm, 1984); Kent et al., pg. 217 in Peptide Chemistry 84, Izumiya, Ed. (Protein Research Foundation, B.H. Osaka, 1985); Merrifield, Science, Vol. 232, pgs. 341-347 (1986); Kent, Ann. Rev. Biochem., Vol. 57, pgs. 957-989 (1988), and references cited in these latter two references.

In solid state synthesis it is most important to eliminate synthesis by-products, which are primarily termination, deletion, or modification peptides. Most side reactions can be eliminated or minimized by use of clean, well characterized resins, clean amino acid derivatives, clean solvents, and the selection of proper coupling and cleavage methods and reaction conditions, e.g. Barany and Merrifield, The Peptides, Cross and Meienhofer, Eds., Vol. 2, pgs 1-284 (Academic Press, New York, 1979). It is important to monitor coupling reactions to determine that they proceed to completion so that deletion peptides missing one or more residues will be avoided. The quantitative ninhydrin reaction is useful for that purpose, Sarin et al. Anal. Biochem, Vol. 117, pg 147 (1981). Na-t-butyloxycarbonyl (t-Boc) - amino acids are used with appropriate side chain protecting groups stable to the conditions of chain assembly but labile to strong acids. After assembly of the protected peptide chain, the protecting groups are removed and the peptide anchoring bond is cleaved by the use of low then high concentrations of anhydrous hydrogen fluoride in the presence of a thioester scavenger, Tam et al., J. Amer. Chem. Soc., Vol. 105, pg. 6442 (1983). Side chain protecting groups used are Asp(OBzl), Glu(OBzl), Ser(Bzl), Thr(Bzl), Lys(Cl-Z), Tyr(Br-Z), Arg(NGTos), Cys(4-MeBzl), and His(ImDNP). (Bzl, benzyl; Tos toluene sulfoxyl; DNP, dinitrophenyl; Im, imidazole; Z, benzyloxgycarbonyl). The remaining amino acids have no side chain protecting groups. For each cycle the tBoc Na protected peptide-resin is exposed to 65 percent trifluoroacetic acid (from Eastman Kodak) (distilled before use) in dichloromethane (DCM), (Mallenckrodt): first for 1 minute then for 13 minutes to remove the Na-protecting group. The peptide-resin is washed in DCM, neutralized twice with 10 percent diisopropylethylamine (DIEA) (Aldrich) in dimethylformamide (DMF) (Applied Biosystems), for 1 minute each. Neutralization is followed by washing with DMF. Coupling is performed with the symmetric anhydride of the amino acid in DMF for 16 minutes. The symmetric anhydride is prepared on the synthesizer by dissolving 2 mmol of amino acid in 6 ml of DCM and adding 1 mmol of dicyclohexycarbodiimide (Aldrich) in 2 ml of DCM. After 5 minutes, the activated amino acid is transferred to a separate vessel and the DCM is evaporated by purging with a continuous stream of nitrogen gas. The DCM is replaced by DMF (6 ml total) at various stages during the purging. After the first coupling, the peptide-resin is washed with DCM, 10 percent DIEA in DCM, and then with DCM. For recoupling, the same amino acid and the activating agent, dicyclohexylcarbodiimide, are transferred sequentially to the reaction vessel. After activation in situ and coupling for 10 minutes, sufficient DMF is added to make a 50 percent DMF-DCM mixture, and the coupling is continued for 15 minutes. Arginine is coupled as a hydroxybenzotriazole (Aldrich) ester in DMF for 60 minutes and then recoupled in the same manner as the other amino acids. Asparagine and glutamine are coupled twice as hydroxybenzotriazole esters in DMF, 40 minutes for each coupling. For all residues, the resin is washed after the second coupling and a sample is automatically taken for monitoring residual uncoupled α-amine by quantitative ninhydrin reaction, Sarin et al. (cited above).

### Anti-TSV polypeptide Antibodies.

The present invention further provides anti-TSV polypeptide antibodies. The antibodies of the present invention include polyclonal, monoclonal, humanized, bispecific, and heteroconjugate antibodies.

Polyclonal Antibodies. The anti-TSV polypeptide antibodies of the present invention may be polyclonal antibodies. Methods of preparing polyclonal antibodies are known to the skilled artisan. Such polyclonal antibodies can be produced in a mammal, for example, following one or more injections of an immunizing agent, and preferably, an adjuvant. Typically, the immunizing agent and/or adjuvant will be injected into the mammal by a series of subcutaneous or intraperitoneal injections. The immunizing agent may include a TSV polypeptide or a fusion protein thereof. It may be useful to conjugate the antigen to a protein known to be immunogenic in the mammal being immunized. Examples of such immunogenic proteins include, but are not limited to, keyhole limpet hemocyanin (KLH), serum albumin, bovine thyroglobulin, and soybean trypsin inhibitor. Adjuvants include, for example, Freund's complete adjuvant and MPL-TDM adjuvant (monophosphoryl Lipid A, synthetic trehalose dicoryno-mycolate). The immunization protocol may be determined by one skilled in the art based on standard protocols or by routine experimentation.

Monoclonal Antibodies. Alternatively, the anti-TSV polypeptide antibodies may be monoclonal antibodies. Monoclonal antibodies may be produced by hybridomas, wherein a mouse, hamster, or other appropriate host animal, is immunized with an immunizing agent to elicit lymphocytes that produce or are capable of producing antibodies that will specifically bind to the immunizing agent [Kohler and Milstein, Nature 256:495 (1975)]. Alternatively, the lymphocytes may be immunized in vitro. The immunizing agent will typically include the TSV polypeptide or a fusion protein thereof. Generally, spleen cells or lymph node cells are used if non-human mammalian sources are desired, or peripheral blood lymphocytes (" PBLs") are used if cells of human origin. The lymphocytes are fused with an immortalized cell line using a suitable fusing agent, such as polyethylene glycol, to produce a hybridoma cell [Goding, *MONOCLONAL ANTIBODIES: PRINCIPLES AND PRACTICE*, Academic Press, pp. 59-103 (1986)]. In general, immortalized cell lines are transformed mammalian cells, for example, myeloma cells of rat, mouse, bovine or human origin. The hybridoma cells are cultured in a suitable culture medium that preferably contains one or more substances that inhibit the growth or survival of unfused, immortalized cells. For example, if the parental cells lack the enzyme hypoxanthine guanine phosphoribosyl transferase (HGPRT), the culture medium for the hybridomas typically will include hypoxanthine, aminopterin, and thymidine (HAT), substances which prevent the growth of HGPRT-deficient cells. Preferred immortalized cell lines are those that fuse efficiently, support stable high level production of antibody, and are sensitive to a medium such as HAT medium. More preferred immortalized cell lines are murine or human myeloma lines, which can be obtained, for example, from the American Type Culture Collection (ATCC), Rockville, MD. Human myeloma and mouse-human heteromyeloma cell lines also have been described for the production of human monoclonal antibodies [Kozbor, J. Zmmunol. 133:3001 (1984); Brodeur et al., Monoclonal Antibody Production Techniques and Applications, Marcel Dekker, Inc., New York, pp. 51-63 (1987)].

The culture medium (supernatant) in which the hybridoma cells are cultured can be assayed for the presence of monoclonal antibodies directed against an TSV polypeptide. Preferably, the binding specificity of monoclonal antibodies present in the hybridoma supernatant is determined by immunoprecipitation or by an in vitro binding assay, such as radio- immunoassay (RIA) or enzyme-linked immunoabsorbent assay (ELISA). Appropriate techniques and assays are known in the art. The binding affinity of the monoclonal antibody can, for example, be determined by the Scatchard analysis of Munson and Pollard, *Anal. Biochem.* 107:220 (1980). After the desired antibody-producing hybridoma cells are identified, the cells may be cloned by limiting dilution procedures and grown by standard methods [Goding, 1986]. Suitable culture media for this purpose include, for example, Dulbecco's Modified Eagle's Medium and RPMI-1640 medium. Alternatively, the hybridoma cells may be grown in vivo as ascites in a mammal. The monoclonal antibodies secreted by selected clones may be isolated or purified from the culture medium or ascites fluid by immunoglobulin purification procedures routinely used by those of skill in the art such as, for example, protein A-Sepharose, hydroxyl-apatite chromatography, gel electrophoresis, dialysis, or affinity chromatography.

The monoclonal antibodies may also be made by recombinant DNA methods, such as those described in U.S. Pat. No. 4,816,567. DNA encoding the monoclonal antibodies of the invention can be isolated from the TSV polypeptide-specific hybridoma cells and sequenced, e.g., by using oligonucleotide probes that are capable of binding specifically to genes encoding the heavy and light chains of murine antibodies. Once isolated, the DNA may be inserted into an expression vector, which is then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for the human heavy and light chain constant domains for the homologous murine sequences [Morrison et al., *Proc. Nat. Acad. Sci.* 81:6851-6855 (1984); Neuberger et al., *Nature* 312:604-608 (1984); Takeda et al., *Nature* 314:452-454 (1985)], or by covalently joining to the immunoglobulin coding sequence all or part of the coding sequence for a non-immunoglobulin polypeptide. The non-immunoglobulin polypeptide can be substituted for the constant domains of an antibody of the invention, or can be substituted for the variable domains of one antigen-combining site of an antibody of the invention to create a chimeric bivalent antibody. The antibodies may also be monovalent antibodies. Methods for preparing monovalent antibodies are well known in the art. For example, in vitro methods are suitable for preparing monovalent antibodies. Digestion of antibodies to produce fragments thereof, particularly, Fab fragments, can be accomplished using routine techniques known in the art.

Antibodies and antibody fragments characteristic of hybridomas of the invention can also be produced by recombinant means by extracting messenger RNA, constructing a cDNA library, and selecting clones which encode segments of the antibody molecule, e.g. Wall et al., Nucleic Acids Research, Vol. 5, pgs. 3113-3128 (1978); Zakut et al., Nucleic Acids Research, Vol. 8, pgs. 3591-3601 (1980); Cabilly et al., Proc. Natl. Acad. Sci., Vol. 81, pgs. 3273-3277 (1984); Boss et al., Nucleic Acids Research, Vol. 12, pgs. 3791-3806 (1984); Amster et al., Nucleic Acids Research, Vol. 8, pgs. 2055-2065 (1980); Moore et al., U.S. Patent 4,642,334; Skerra et al, Science, Vol. 240, pgs. 1038-1041(1988); and Huse et al, Science, Vol. 246, pgs. 1275-1281 (1989). In particular, such techniques can be used to produce interspecific monoclonal antibodies, wherein the binding region of one species is combined with non-binding region of the antibody of another species to reduce immunogenicity, e.g. Liu et al., Proc. Natl. Acad. Sci., Vol. 84, pgs. 3439-3443 (1987).

Both polyclonal and monoclonal antibodies can be screened by ELISA. As in other solid phase immunoassays, the test is based on the tendency of macromolecules to adsorb nonspecifically to plastic. The irreversibility of this reaction, without loss of immunological activity, allows the formation of antigen-antibody complexes with a simple separation of such complexes from unbound material. To titrate antipeptide serum, peptide conjugated to a carrier different from that used in immunization is adsorbed to the wells of a 96-well microtiter plate. The adsorbed antigen is then allowed to react in the wells with dilutions of anti-peptide serum. Unbound antibody is washed away, and the remaining antigen-antibody complexes are allowed to react with antibody specific for the IgG of the immunized animal. this second antibody is conjugated to an enzyme such as alkaline phosphatase. A visible colored reaction product produced when the enzyme substrate is added indicates which wells have bound antipeptide antibodies. The use of spectrophotometer readings allows better quantification of the amount of peptide-specific antibody bound. High-titer antisera yield a linear titration curve between 10⁻³ and 10⁻⁵ dilutions.

TSV peptide antibodies. The invention includes peptides derived from TSV polypeptide, and immunogens comprising conjugates between carriers and peptides of the invention. The term immunogen as used herein refers to a substance which is capable of causing an immune response. The term carrier as used herein refers to any substance which when chemically conjugated to a peptide of the invention permits a host organism immunized with the resulting conjugate to generate antibodies specific for the conjugated peptide. Carriers include red blood cells, bacteriophages, proteins, or synthetic particles such as agarose beads. Preferably, carriers are proteins, such as serum albumin, gamma-globulin, keyhole limpet hemocyanin, thyroglobulin, ovalbumin, fibrinogen, or the like.

The general technique of linking synthetic peptides to a carrier is described in several references, e.g. Walter and Doolittle, "Antibodies Against Synthetic Peptides," in Setlow et al., eds., Genetic Engineering, Vol. 5, pgs. 61-91 (Plenum Press, N.Y., 1983); Green et al. Cell, Vol. 28, pgs. 477-487 (1982); Lerner et al., Proc. Natl. Acad. Sci., Vol. 78, pgs. 3403-3407 (1981); Shimizu et al., U.S. Patent 4,474,754; and Ganfield et al., U.S. Patent 4,311,639. Accordingly, these references are incorporated by reference. Also, techniques employed to link haptens to carriers are essentially the same as the above-referenced techniques, e.g. chapter 20 in Tijsseu Practice and Theory of Enzyme Immunoassays (Elsevier, New York, 1985). The four most commonly used schemes for attaching a peptide to a carrier are (1) glutaraldehyde for amino coupling, e.g. as disclosed by Kagan and Glick, in Jaffe and Behrman, eds. Methods of Hormone Radioimmunoassay, pgs. 328-329 (Academic Press, N.Y., 1979), and Walter et al. Proc. Natl. Acad. Sci., Vol. 77, pgs. 5197-5200 (1980); (2) water-soluble carbodiimides for carboxyl to amino coupling, e.g. as disclosed by Hoare et al., J. Biol. Chem., Vol. 242, pgs. 2447-2453 (1967); (3) bis-diazobenzidine (DBD) for tyrosine to tyrosine sidechain coupling, e.g. as disclosed by Bassiri et al., pgs. 46-47, in Jaffe and Behrman, eds. (cited above), and Walter et al. (cited above); and (4) maleimidobenzoyl-N-hydroxysuccinimide ester (MBS) for coupling cysteine (or other sulfhydryls) to amino groups, e.g. as disclosed by Kitagawa et al., J. Biochem. (Tokyo), Vol. 79, pgs. 233-239 (1976), and Lerner et al. (cited above). A general rule for selecting an appropriate method for coupling a given peptide to a protein carrier can be stated as follows: the group involved in attachment should occur only once in the sequence, preferably at the appropriate end of the segment. For example, BDB should not be used if a tyrosine residue occurs in the main part of a sequence chosen for its potentially antigenic character. Similarly, centrally located lysines rule out the glutaraldehyde method, and the occurrences of aspartic and glutamic acids frequently exclude the carbodiimide approach. On the other hand, suitable residues can be positioned at either end of chosen sequence segment as attachment sites, whether or not they occur in the "native" protein sequence. Internal segments, unlike the amino and carboxy termini, will differ significantly at the "unattached end" from the same sequence as it is found in the native protein where the polypeptide backbone is continuous. The problem can be remedied, to a degree, by acetylating the α-amino group and then attaching the peptide by way of its carboxy terminus. The coupling efficiency to the carrier protein is conveniently measured by using a radioactively labeled peptide, prepared either by using a radioactive amino acid for one step of the synthesis or by labeling the completed peptide by the iodination of a tyrosine residue. The presence of tyrosine in the peptide also allows one to set up a sensitive radioimmune assay, if desirable. Therefore, tyrosine can be introduced as a terminal residue if it is not part of the peptide sequence defined by the native polypeptide.

Preferred carriers are proteins, and preferred protein carriers include bovine serum albumin, myoglobulin, ovalbumin (OVA), keyhole limpet hemocyanin (KLH), or the like. Peptides can be linked to KLH through cysteines by MBS as disclosed by Liu et al., Biochemistry, Vol. 18, pgs. 690-697 (1979). The peptides are dissolved in phosphate-buffered saline (pH 7.5), 0.1 M sodium borate buffer (pH 9.0) or 1.0 M sodium acetate buffer (pH 4.0). The pH for the dissolution of the peptide is chosen to optimize peptide solubility. The content of free cysteine for soluble peptides is determined by Ellman's method, Ellman, Arch. Biochem. Biophys., Vol. 82, pg. 7077 (1959). For each peptide, 4 mg KLH in 0.25 ml of 10 mM sodium phosphate buffer (pH 7.2) is reacted with 0.7 mg MBS (dissolved in dimethyl formamide) and stirred for 30 min at room temperature. The MBS is added dropwise to ensure that the local concentration of formamide is not too high, as KLH is insoluble in >30% formamide. The reaction product, KLH-MBS, is then passed through Sephadex G-25 equilibrated with 50 mM sodium phosphate buffer (pH 6.0) to remove free MBS, KLH recovery from peak fractions of the column eluate (monitored by OD280) is estimated to be approximately 80%. KLH-MBS is then reacted with 5 mg peptide dissolved 25 in 1 ml of the chosen buffer. The pH is adjusted to 7-7.5 and the reaction is stirred for 3 hr at room temperature. Coupling efficiency is monitored with radioactive peptide by dialysis of a sample of the conjugate against phosphate-buffered saline, and ranged from 8% to 60%. Once the peptide-carrier conjugate is available polyclonal or monoclonal antibodies are produced by standard techniques, e.g. as disclosed by Campbell, Monoclonal Antibody Technology (Elsevier, New York, 1984); Hurrell, ed. Monoclonal Hybridoma Antibodies: Techniques and Applications (CRC Press, Boca Raton, FL, 1982); Schreier et al. Hybridoma Techniques (Cold Spring Harbor Laboratory, New York, 1980); U.S. Patent 4,562,003; or the like. In particular, U.S. Patent 4,562,003 is incorporated by reference.

Humanized Antibodies. The anti-TSV polypeptide antibodies of the invention may further comprise humanized antibodies or human antibodies. The term "humanized antibody" refers to humanized forms of non-human (e.g., murine) antibodies that are chimeric antibodies, immunoglobulin chains or fragments thereof (such as Fv, Fab, Fab', F(ab'), or other antigen-binding partial sequences of antibodies) which contain some portion of the sequence derived from non-human antibody. Humanized antibodies include human immunoglobulins in which residues from a complementary determining region (CDR) of the human immunoglobulin are replaced by residues from a CDR of a non-human species such as mouse, rat or rabbit having the desired binding specificity, affinity and capacity. In general, the humanized antibody will comprise substantially all of at least one, and generally two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., *Nature* 321:522-525 (1986) and Presta, *Cuvv. Op. Stvuct. Biol. 2:593-596* (1992)]. Methods for humanizing non-human antibodies are well known in the art. Generally, a humanized antibody has one or more amino acids introduced into it from a source which is non-human in order to more closely resemble a human antibody, while still retaining the original binding activity of the antibody. Methods for humanization of antibodies are further detailed in Jones et al., *Nature* 321:522-525 (1986); Riechmann et al., *Nature* 332:323-327 (1988); and Verhoeyen et al., *Science* 239:1534-1536 (1988). Such "humanized" antibodies are chimeric antibodies in that substantially less than an intact human variable domain has been substituted by the corresponding sequence from a non-human species.

Heteroconjugate Antibodies. Heteroconjugate antibodies which comprise two covalently joined antibodies, are also within the scope of the present invention. Heteroconjugate antibodies may be prepared in vitro using known methods in synthetic protein chemistry, including those involving crosslinking agents. For example, immunotoxins may be prepared using a disulfide exchange reaction or by forming a thioether bond.

Bispecific Antibodies. Bispecific antibodies have binding specificities for at least two different antigens. Such antibodies are monoclonal, and preferably human or humanized. One of the binding specificities of a bispecific antibody of the present invention is for a TSV polypeptide, and the other one is preferably for a cell-surface protein or receptor or receptor subunit. Methods for making bispecific antibodies are known in the art, and in general, the recombinant production of bispecific antibodies is based on the co-expression of two immunoglobulin heavy-chain/light-chain pairs in hybridoma cells, where the two heavy chains have different specificities [Milstein and Cuello, *Nature 305:537-539* (1983)]. Given that the random assortment of immunoglobulin heavy and light chains results in production of potentially ten different antibody molecules by the hybridomas, purification of the correct molecule usually requires some sort of affinity purification, e.g. affinity chromatography.

Antibody antagonists. Preferably, antagonists of the invention are derived from antibodies specific for TSV polypeptide. More preferably, the antagonists of the invention comprise fragments or binding compositions specific for TSV polypeptide. Antibodies comprise an assembly of polypeptide chains linked together by disulfide bridges. Two major polypeptide chains, referred to as the light chain and the heavy chain, make up all major structural classes (isotypes) of antibody. Both heavy chains and light chains are further divided into subregions referred to as variable regions and constant regions. Heavy chains comprise a single variable region and three different constant regions, and light chains comprise a single variable region (different from that of the heavy chain) and a single constant region (different from those of the heavy chain). The variable regions of the heavy chain and light chain are responsible for the antibody's binding specificity. As used herein, the term "heavy chain variable region" means a polypeptide (1) which is from 110 to 125 amino acids in length, and (2) whose amino acid sequence corresponds to that of a heavy chain of a monoclonal antibody of the invention, starting from the heavy chain's N-terminal amino acid. Likewise, the term "light chain variable region" means a polypeptide (1) which is from 95 to 115 amino acids in length, and (2) whose amino acid sequence corresponds to that of a light chain of a monoclonal antibody of the invention, starting from the light chain's N-terminal amino acid. As used herein the term "monoclonal antibody" refers to homogeneous populations of immunoglobulins which are capable of specifically binding to TSV polypeptide. As used herein the term "binding composition" means a composition comprising two polypeptide chains (1) which, when operationally associated, assume a conformation having high binding affinity for TSV polypeptide, and (2) which are derived from a hybridoma producing monoclonal antibodies specific for TSV polypeptide. The term "operationally associated" is meant to indicate that the two polypeptide chains can be positioned relative to one another for binding by a variety of means, including by association in a native antibody fragment, such as Fab or Fv, or by way of genetically engineered cysteine-containing peptide linkers at the carboxyl termini. Normally, the two polypeptide chains correspond to the light chain variable region and heavy chain variable region of a monoclonal antibody specific for TSV polypeptide. Preferably, antagonists of the invention are derived from monoclonal antibodies specific for TSV polypeptide. Monoclonal antibodies capable of blocking, or neutralizing, TSV polypeptide are selected by their ability to inhibit TSV polypeptide-induced effects.

The use and generation of fragments of antibodies is also well known, e.g. Fab fragments: Tijssen, Practice and Theory of Enzyme Immunoassays (Elsevier, Amsterdam, 1985); and Fv fragments: Hochman et al. Biochemistry, Vol. 12, pgs. 1130-1135 (1973), Sharon et al., Biochemistry, Vol. 15, pgs. 1591-1594 (1976) and Ehrlich et al., U.S. Patent 4,355,023; and antibody half molecules: Auditore- Hargreaves, U.S. Patent 4,470,925.

### Purification and Pharmaceutical Compositions

When polypeptides of the present invention are expressed in soluble form, for example as a secreted product of transformed yeast or mammalian cells, they can be purified according to standard procedures of the art, including steps of ammonium sulfate precipitation, ion exchange chromatography, gel filtration, electrophoresis, affinity chromatography, and/or the like, e.g. "Enzyme Purification and Related Techniques," Methods in Enzymology, 22:233-577 (1977), and Scopes, R., Protein Purification: Principles and Practice (Springer-Verlag, New York, 1982) provide guidance in such purifications. Likewise, when polypeptides of the invention are expressed in insoluble form, for example as aggregates, inclusion bodies, or the like, they can be purified by standard procedures in the art, including separating the inclusion bodies from disrupted host cells by centrifugation, solublizing the inclusion bodies with chaotropic and reducing agents, diluting the solubilized mixture, and lowering the concentration of chaotropic agent and reducing agent so that the polypeptide takes on a biologically active conformation. The latter procedures are disclosed in the following references, which are incorporated by reference: Winkler et al, Biochemistry, 25: 4041-4045 (1986); Winkler et al, Biotechnology, 3: 992-998 (1985); Koths et al, U.S. patent 4,569,790; and European patent applications 86306917.5 and 86306353.3.

As used herein "effective amount" means an amount sufficient to ameliorate a symptom of an autoimmune condition. The effective amount for a particular patient may vary depending on such factors as the state of the condition being treated, the overall health of the patient, method of administration, the severity of side-effects, and the like. Generally, TSV polypeptide is administered as a pharmaceutical composition comprising an effective amount of TSV polypeptide and a pharmaceutical carrier. A pharmaceutical carrier can be any compatible, non-toxic substance suitable for delivering the compositions of the invention to a patient. Generally, compositions useful for parenteral administration of such drugs are well known, e.g. Remington's Pharmaceutical Science, 15th Ed. (Mack Publishing Company, Easton, PA 1980). Alternatively, compositions of the invention may be introduced into a patient's body by implantable or injectable drug delivery system, e.g. Urquhart et al., Ann. Rev. Pharmacol. Toxicol., Vol. 24, pgs. 199-236 (1984); Lewis, ed. Controlled Release of Pesticides and Pharmaceuticals (Plenum Press, New York, 1981); U.S. patent 3,773,919; U.S. patent 3,270,960; and the like.

When administered parenterally, the TSV polypeptide is formulated in a unit dosage injectable form (solution, suspension, emulsion) in association with a pharmaceutical carrier. Examples of such carriers are normal saline, Ringer's solution, dextrose solution, and Hank's solution. Nonaqueous carriers such as fixed oils and ethyl oleate may also be used. A preferred carrier is 5% dextrose/saline. The carrier may contain minor amounts of additives such as substances that enhance isotonicity and chemical stability, e.g., buffers and preservatives. The TSV polypeptide is preferably formulated in purified form substantially free of aggregates and other proteins at a concentration in the range of about 5 to 20 µg/ml. Preferably, TSV polypeptide is administered by continuous infusion so that an amount in the range of about 50-800 µg is delivered per day (i.e. about 1-16 µg/kg/day). The daily infusion rate may be varied based on monitoring of side effects, such as blood cell counts, body temperature, and the like.

TSV polypeptide can be purified from culture supernatants of mammalian cells transiently transfected or stably transformed by an expression vector carrying an TSV polypeptide gene. Preferably, TSV polypeptide is purified from culture supernatants of COS 7 cells transiently transfected by the pcD expression vector. Transfection of COS 7 cells with pcD proceeds as follows: One day prior to transfection, approximately 10⁶ COS 7 monkey cells are seeded onto individual 100 mm plates in Dulbecco's modified Eagle medium (DME) containing 10% fetal calf serum and 2 mM glutamine. To perform the transfection, the medium is aspirated from each plate and replaced with 4 ml of DME containing 50 mM Tris.HCl pH 7.4, 400 mg/ml DEAE-Dextran and 50 µg of plasmid DNA. The plates are incubated for four hours at 37°C, then the DNA-containing medium is removed, and the plates are washed twice with 5 ml of serum-free DME. DME is added back to the plates which are then incubated for an additional 3 hrs at 37°C. The plates are washed once with DME, after which DME containing 4% fetal calf serum, 2 mM glutamine, penicillin (100 U/L) and streptomycin (100 µg/L) at standard concentrations is added. The cells are then incubated for 72 hrs at 37°C, after which the growth medium is collected for purification of TSV polypeptide. Alternatively, transfection can be accomplished by electroporation as described in the examples. Plasmid DNA for the transfections is obtained by growing pcD(SRα), or like expression vector, containing the TSV polypeptide cDNA insert in E. coli MC1061, described by Casadaban and Cohen, J. Mol. Biol., Vol. 138, pgs. 179-207 (1980), or like organism. The plasmid DNA is isolated from the cultures by standard techniques, e.g. Sambrook et al., Molecular Cloning: A Laboratory Manual, Second Edition (Cold Spring Harbor Laboratory, New York, 1989) or Ausubel et al (1990, cited above).

When the antagonists of the inventions are derived from antibodies, they are normally administered parenterally, preferably intravenously. Since such protein or peptide antagonists may be immunogenic they are preferably administered slowly, either by a conventional IV administration set or from a subcutaneous depot, e.g. as taught by Tomasi et al, U.S. patent 4,732,863. When administered parenterally, the antibodies and/or fragments are formulated in a unit dosage injectable form in association with a pharmaceutical carrier, as described above. The antibody is preferably formulated in purified form substantially free of aggregates, other proteins, endotoxins, and the like, at concentrations of about 5 to 30 mg/ml, preferably 10 to 20 mg/ml. Preferably, the endotoxin levels are less than 2.5 EU/ml.

Selecting an administration regimen for an antagonist depends on several factors, including the serum turnover rate of the antagonist, the serum level of TSV polypeptide associated with the disorder being treated, the immunogenicity of the antagonist, the accessibility of the target TSV polypeptide (e.g. if non-serum TSV polypeptide is to be blocked), the relative affinity of TSV polypeptide to its receptor(s) versus TSV polypeptide to the antagonist, and the like. Preferably, an administration regimen maximizes the amount of antagonist delivered to the patient consistent with an acceptable level of side effects. Accordingly, the amount of antagonist delivered depends in part on the particular antagonist and the severity of the condition being treated. Guidance in selecting appropriate doses is found in the literature on therapeutic uses of antibodies, e.g. Bach et al., chapter 22, in Ferrone et al., eds., Handbook of Monoclonal Antibodies (Noges Publications, Park Ridge, NJ, 1985); and Russell, pgs. 303-357, and Smith et al., pgs. 365-389, in Haber et al., eds. Antibodies in Human Diagnosis and Therapy (Raven Press, New York, 1977). Preferably, whenever the antagonist comprises monoclonal antibodies or Fab-sized fragments thereof (including binding compositions), the dose is in the range of about 1-20 mg/kg per day. More preferably the dose is in the range of about 1-10 mg/kg per day.

### Example 1

### Chentical Synthesis of TSV polypeptide

In this example, a polypeptide having the sequence of Fig. 1 is synthesized by standard solid phase peptide synthesis. Clean, well characterized resins, clean amino acid derivatives, clean solvents are used in all operations, e.g. Barany and Merrifield, The Peptides, Cross and Meienhofer, Eds., Vol. 2, pgs 1-284 (Academic Press, New York, 1979). Coupling reactions are monitored to determine that they proceed to completion so that deletion peptides missing one or more residues will be avoided. The quantitative ninhydrin reaction is useful for that purpose, Sarin et al. Anal. Biochem, Vol. 117, pg 147 (1981). Na-t-butyloxycarbonyl (t-Boc) - amino acids are used with appropriate side chain protecting groups stable to the conditions of chain assembly but labile to strong acids. After assembly of the protected peptide chain, the protecting groups are removed and the peptide anchoring bond is cleaved by the use of low then high concentrations of anhydrous hydrogen fluoride in the presence of a thioester scavenger, Tam et al., J. Amer. Chem. Soc., Vol. 105, pg. 6442 (1983). Side chain protecting groups used are Asp(OBzl), Glu(OBzl), Ser(Bzl), Thr(Bzl), Lys(Cl-Z), Tyr(Br-Z), Arg(NGTos), Cys(4-MeBzl), and His(ImDNP). (Bzl, benzyl; Tos toluene sulfoxyl; DNP, dinitrophenyl; Im, imidazole; Z, benzyloxgycarbonyl). The remaining amino acids have no side chain protecting groups. For each cycle the tBoc Na protected peptide-resin is exposed to 65 percent trifluoroacetic acid (from Eastman Kodak) (distilled before use) in dichloromethane (DCM), (Mallenckrodt): first for 1 minute then for 13 minutes to remove the Na-protecting group. The peptide-resin is washed in DCM, neutralized twice with 10 percent diisopropylethylamine (DIEA) (Aldrich) in dimethylformamide (DMF) (Applied Biosystems), for 1 minute each. Neutralization is followed by washing with DMF. Coupling is performed with the symmetric anhydride of the amino acid in DMF for 16 minutes. The symmetric anhydride is prepared on the synthesizer by dissolving 2 mmol of amino acid in 6 ml of DCM and adding 1 mmol of dicyclohexycarbodiimide (Aldrich) in 2 ml of DCM. After 5 minutes, the activated amino acid is transferred to a separate vessel and the DCM is evaporated by purging with a continuous stream of nitrogen gas. The DCM is replaced by DMF (6 ml total) at various stages during the purging. After the first coupling, the peptide-resin is washed with DCM, 10 percent DIEA in DCM, and then with DCM. For recoupling, the same amino acid and the activating agent, dicyclohexylcarbodiimide, are transferred sequentially to the reaction vessel. After activation in situ and coupling for 10 minutes, sufficient DMF is added to make a 50 percent DMF-DCM mixture, and the coupling is continued for 15 minutes. Arginine is coupled as a hydroxybenzotriazole (Aldrich) ester in DMF for 60 minutes and then recoupled in the same manner as the other amino acids. Asparagine and glutamine are coupled twice as hydroxybenzotriazole esters in DMF, 40 minutes for each coupling. For all residues, the resin is washed after the second coupling and a sample is automatically taken for monitoring residual uncoupled α-amine by quantitative ninhydrin reaction, Sarin et al. (cited above).

### Example 2

### Monoclonal Antibodies Specific for TSV polypeptide

A male Lewis rat is immunized with semi-purified preparations of chemically synthesized TSV polypeptide. The rat is first immunized with approximately 50 µg of TSV polypeptide in Freund's Complete Adjuvant, and boosted twice with the same amount of material in Freund's Incomplete Adjuvant. Test bleeds are taken. The animal is given a final boost of 25 µg in phosphate-buffered saline, and four days later the spleen is obtained for fusion.

Approximately 3 x 10⁸ rat splenocytes are fused with an equal number of P3X63-AG8.653 mouse myeloma cells (available from the ATCC under accession number CRL 1580). 3840 microtiter plate wells are seeded at 5.7 x 10⁴ parental myeloma cells per well. Standard protocols for the fusion and subsequent culturing of hybrids are followed, e.g. as described by Chretien et al, J. Immunol. Meth., Vol. 117, pgs. 67-81 (1989). 12 days after fusion supernatants are harvested and screened by indirect ELISA on PVC plates coated with chemically synthesized TSV polypeptide.

The descriptions of the foregoing embodiments of the invention have been presented for purpose of illustration and description. They are not intended to be exhaustive or to limit the invention to the precise forms disclosed, and obviously many modifications and variations are possible in light of the above teaching. The embodiments were chosen and described in order to best explain the principles of the invention to thereby enable others skilled in the art to best utilize the invention in various embodiments and with various modifications as are suited to the particular use contemplated. It is intended that the scope of the invention be defined by the claims appended hereto.

## Claims

1. An isolated polypeptide having an amino acid sequence selected from the group consisting of:
(a) an amino acid sequence as depicted in SEQ ID NO: 3;
(b) an amino acid sequence having at least 97% identity with SEQ ID NO. 3; and
(c) a fragment of (a) or (b).

2. The polypeptide of claim 1 fused to a heterologous amino acid sequence.

3. An antibody or fragment thereof that specifically binds to the polypeptide of claim 1.

4. A pharmaceutical composition comprising the polypeptide of claim 1 or 2 or the antibody or fragment thereof of claim 3 and a pharmaceutically acceptable carrier.

5. A diagnostic composition comprising the antibody of claim 3.

6. A method of binding a TSV polypeptide, said method comprising:
(a) providing a polypeptide of claim 1; and
(b) bringing said polypeptide into contact with an antibody that binds said polypeptide.

7. A diagnostic method for the detection of a TSV polypeptide in a sample comprising the step of contacting the antibody of claim 3 with said sample.

8. An isolated polynucleotide having a nucleotide sequence that encodes the polypeptide of claim 1.

9. The polynucleotide of claim 8, wherein said polynucleotide is operably linked to a promoter.

10. A vector comprising the polynucleotide of claim 8.

11. A host cell comprising the vector of claim 10.

12. A method of making a TSV polypeptide, said method comprising:
(a) providing a population of host cells comprising a recombinant polynucleotide encoding a TSV polypeptide of claim 1; and
(b) culturing said population of host cells under conditions conducive to the expression of said recombinant polynucleotide;
whereby said polypeptide is produced within said population of host cells.

13. The method of claim 12, further comprising purifying said polypeptide from said population of cells.

14. Use of the antibody of claim 3 for the preparation of a pharmaceutical composition for treating a TSV polypeptide related disorder.
